# EUROPEAN PATENT APPLICATION

(11) **EP 1 724 280 A1**
(43) Date of publication of application: **22.11.2006**
(21) Application number: 05717200.9
(22) Date of filing: 07.03.2005
(51) Int. Cl.: C07K 7/06, C07K 7/08, A61K 38/08, A61K 38/10, A61K 47/42

(54) **USE OF PEPTIDES AS PENETRATING CELL CARRIERS**

(30) Priority: 08.03.2004 ES 200400653
(71) Applicant: Universidad de Barcelona, 08028 Barcelona (ES)
(72) Inventor: GIRALT LLED , Ernest, E-08028 Barcelona (ES); FERN NDEZ CARNEADO, Jimena, E-08028 Barcelona (ES)
(74) Representative: Fisher, Adrian John
(86) International application number: PCT/ES2005/000116
(87) International publication number: WO 2005/087795

(57) **Abstract**

The invention relates to compounds having formula (I) or the biologically- or pharmaceutically-acceptable salts thereof which are used as penetrating cell carriers. According to formula (I), Val is L-Val or D-Val; Arg is L-Arg, D-Arg or L-(N-methyl)Arg; Pro is L-Pro or D-Pro; Leu is L-Leu or D-Leu; x is an integer of between 1 and 20, preferably 3; L₁ and L₂ are chemical linkers; M₁ and M₂ are pharmaceutically- and/or biologically-active groups; and the linkage between L₁ and M₁ and the linkage between L₂ and M₂ are of any known chemical nature, including covalent and ionic. Compared to other previously-described carrier peptides, said novel family offers many advantages including its non-viral origin, amphipathic character, water solubility and the absence of a cytotoxic effect at high concentrations. M₁-L₁-(Val-Arg-Leu-Pro-Pro-Pro)x-L₂-M₂ (I)

## Description

This invention relates to the fields of medicine, research, diagnostics, and cosmetics, and specifically to new peptides as cell penetrating carriers for the delivery of compounds into cells.

### BACKGROUND ART

The poor permeability of the cell membrane to outside agents is a significant limitation for research and particularly for medicine in the development of many drugs. Efficient cellular uptake of many chemical agents is still a challenge.

Research in this field has taken several approaches. A first approach involves the use of penetration enhancers such as chellating agents, surfactants and non-surfactants, bile salts and fatty acids, that have good effects on penetration but also safety concerns. A second approach involves the use of technologies based on liposomes, viral vectors and microinjection, but it still has little success.

The ability of certain peptides to cross eukaryotic cell membranes is of interest in this field. In recent years, this interest has led to a rapid development of peptide carriers for the delivery of anti-tumoral, anti-viral or antibiotic drugs, which otherwise would be unable to cross the cell membrane and reach their therapeutic target. The use of peptide carriers has the advantage of allowing accessible synthesis and high flexibility for modification when attaching peptides or small drug molecules as cargoes. The ability of a wide variety of short peptides to act as carriers for the delivery of other peptides, proteins or oligonucleotides inside the cell has been demonstrated. The most prominent examples are human calcitonin (hCT), fragments of transduction proteins domains such as VP22 (cf. S.R. Schwarze et al., "Protein transduction: unrestricted delivery into all cells?", Trend in Cell Biol. 2000, vol. 10, pp. 290-5), HIV transactivator of transcription, also known as "Tat" (cf. M. Silhol et al., "Different mechanisms for cellular internalization of the HIV-1 Tat-derived cell penetrating peptide and recombinant proteins fused to Tat", Eur. J. Biochem. 2002, vol. 269, pp. 494-501) or Antennapedia, also known as "Antp" (cf.

D.J. Dunican et. al., "Designing cell-permeant phosphopeptides to modulate intracellular Signaling pathways", Biopolymers Pept. Sci. 2001, vol. 60, pp. 45-60), arginine-rich peptides (cf. J.B. Rothbard et al., "Arginine-rich molecular transporters for drug delivery: role of backbone spacing in cellular uptake", J. Med. Chem. 2002, vol. 45, pp. 3612-18; N. Emi et al., "Gene transfer mediated by polyarginine requires a formation of big carrier-complex of DNA aggregate", Biochem. and Biophys. Res. Commun. 1997, vol 231, pp. 421-4), β-peptides, peptoids and loligomers (branched peptides rich in Lys). All these cell penetrating peptides have proven to be valuable in the delivery of biologically active cargoes to the cytoplasm and nucleus. However, the main drawback of these peptides is that they are cytotoxic at moderate and high concentrations. Furthermore, laboratory handling associated with the use of these peptides is difficult.

Several years ago peptides of general formula (Val-His-Leu-Pro-Pro-Pro)ₓ, (using three-letter amino acid codes) with x = 2-8 were synthesized using a solid phase convergent approach (cf. I. Dalcol et al., "Convergent solid phase peptide synthesis: an efficient approach to the synthesis of highly repetitive domains", J. Org. Chem. 1995, vol. 60, pp. 7575-81). But the ability of these peptides to form complexes with drugs or other compounds and to act as carriers capable of translocating these compounds has not been described.

### SUMMARY OF THE INVENTION

Inventors provide a new family of peptides which have the ability to cross cell membranes and therefore, to act as cell penenatring carriers of drugs and/or other molecules. In particular, these peptides have a high penetration efficiency and do not have cytotoxic effects at high concentrations.

Thus, an aspect of the present invention relates to compounds of formula (I) or pharmaceutically or biologically acceptable salts thereof, wherein Val is L-Val or D-Val; Arg is L-Arg, D-Arg or L-(N-methyl)Arg; Pro is L-Pro or D-Pro; Leu is L-Leu or D-Leu; x is an integer from 1 to 20; L₁ and L₂ are chemical linkers, which are identical or different, present or absent; M₁ and M₂ are pharmacologically and/or biologically active moieties, which are identical or different, present or absent; and the bond between L₁ and M₁ and the bond between L₂ and M₂ are of any of the known chemical natures, including covalent and ionic.

M₁-L₁-(Val-Arg-Leu-Pro-Pro-Pro)ₓ-L₂-M₂ (I)

N-methylation in (N-methyl)Arg occurs in the framework amino group of the Arg amino acid, not in the side-chain amino group. Amino acids making up peptide sequence can have L or D-configuration. Synthesis with D-configuration amino acids is more expensive but it is useful in order to avoid peptide degradation by proteases. Inclusion of at least one D-amino acid in peptide sequence is enough to achieve proteases resistance and therefore, to make peptides usable for medical applications.

The compounds of the invention may be synthesized using e.g. solid phase, liquid phase, or combinational synthesis techniques thereof, as it is known to a person skilled in the art. Alternatively, peptides may be synthesized from a nucleic acid coding for the peptide. This nucleic acid may be introduced to a cell system and expressed to produce large scale quantities of the peptide.

The compounds according to the present invention represented by formula (I) can also be obtained by known methods as pharmaceutically y/or biologically acceptable salts, such as the sodium salt, the potassium salt, the calcium salt, the magnesium salt, and acid addition salts. Examples of the latter salts include salts of inorganic acids (e.g. hydrochloric acid, sulphuric acid and phosphoric acid) and of organic acids (e.g. acetic acid, propionic acid, citric acid, tartaric acid, malic acid and methanesulfonic acid).

Chemical linkers included in formula (I) can be aliphatic chains (e.g. amino acids and polyethylene glycol) or aromatic chains. In a particular embodiment of the invention linkers are independently selected from peptide sequences up to 10 amino acid residues. A wide range of moieties having pharmacological and/or biological activity can be included in formula (I). Moieties can be active pharmaceutical ingredients, nucleic acids (e.g. oligonucleotides, double strand DNA, single strand DNA, circular DNA, RNA and signal interfering RNA -siRNA-), peptide nucleic acids (PNAs), nanoparticles, antibodies and radioactive and fluorescent dyes or probes (e.g. carboxyfluorescein and derivatives, lucifer yellow, rhodamine, and texas red). Moieties can also be peptides, proteins, carbohydrates, lipids or sterols. Examples of active pharmaceutical ingredients are dopamina, doxorubicin, daunomycin, paclitaxel and therapeutic peptides and proteins. A composition for delivery into cells can comprise more than one type of molecule, for example, two different DNA sequences.

It is well known to those skilled in the art how to chemically bind a peptide to a moiety and/or to a linker. Thus, the bond between L₁ and M₁ and the bond between L₂ and M₂ may be of any of the known chemical natures, depending on the nature of L₁, L₂, M₁, M₂ and on the nature of the peptide, including covalent and ionic bonds. It is important that the selected chemical bond maintain activity of the corresponding moiety.

In an embodiment of the invention, parameter x of formula (I) is from 1 to 10, more especifically from 1 to 3 and preferably 3. In another embodiment, L₁, L₂, M₁ and M₂ are all absent and consequently, the compound of formula (I) is (Val-Arg-Leu-Pro-Pro-Pro)ₓ, wherein x is preferably 3. In a particular embodiment, compounds have a sequence selected from the group consisting of SEQ ID NO: 1-6.

Another aspect of the invention relates to the use of peptides of the present invention as cell penetrating carriers. Compared with other previously described carrier peptides, peptides of the invention present several advantages, including their non-viral origin, their amphipathic character, their solubility in water and the absence of a cytotoxic effect at high concentrations (more than 1000 µM). Other peptides such as Antp or Tat are cytotoxic at concentrations of 50 µM.

The term "carrier" means herein any substance used to support or to transport another substance. In this invention 5(6)-carboxyfluorescein is used as a moiety to illustrate the potential of new peptides to act as cell penetrating carriers. In the specific example included, 5(6)-carboxyfluorescein is delivered inside HeLa human cell line. However, peptides of the invention are useful for other types of eukaryotic cells and also for prokaryotic cells. In a particular application, cell penetrating carriers of the invention are usable to act as transfection agents. The term "transfection" means herein the process of inserting foreign DNA into a culture of eukaryotic cells by exposing them to naked DNA (analogous to transformation in prokariotic cells).

Another aspect of the invention refers to pharmaceutical compositions comprising a therapeutically effective amount of a compound of the invention, together with appropriate amounts of pharmaceutical excipients. And in the same way, the invention provides cosmetic compositions comprising a cosmetically effective amount of a compound of the invention, together with appropriate amounts of cosmetic excipients. The invention involves the use of a single peptide or a mixture thereof. The person skilled in the art will choose appropriate administration via for these compositions, for example oral, parenteral, nasal or topical.

Throughout the description and claims the word "comprise" and its variations are not intended to exclude other technical features, additives, components, or steps. The abstract of this application is incorporated herein as reference. Additional objects, advantages and features of the invention will become apparent to those skilled in the art upon examination of the description or may be learned by practice of the invention. The following particular embodiments, drawings and sequences listing are provided by way of illustration, and they are not intended to be limiting of the present invention.

### DESCRIPTION OF THE DRAWINGS

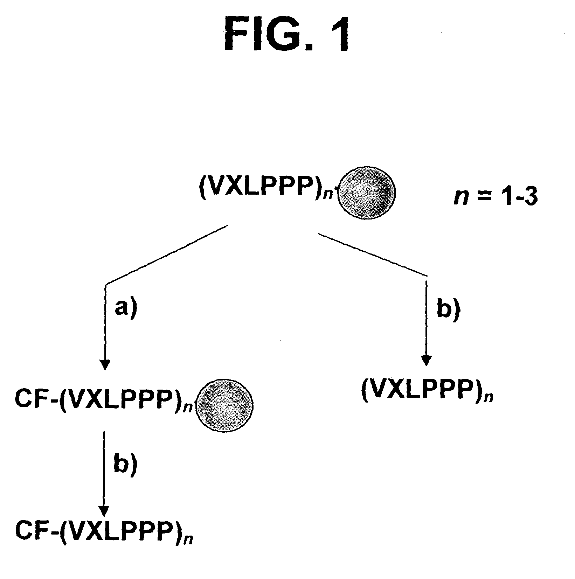

FIG. 1. shows an scheme of synthesis and carboxyfluoresceination reaction on solid support (resin = 2-chlorotrityl resin); a) 5(6)-carboxyfluorescein (5 eq), PyAOP (5 eq), HOAt (5 eq), DIEA (10 eq), DMF, 1 h 30 min, r.t., b) 95% TFA, 2.5% TIS, 2.5% water (15 min-1 h 30 min). R means "resin".

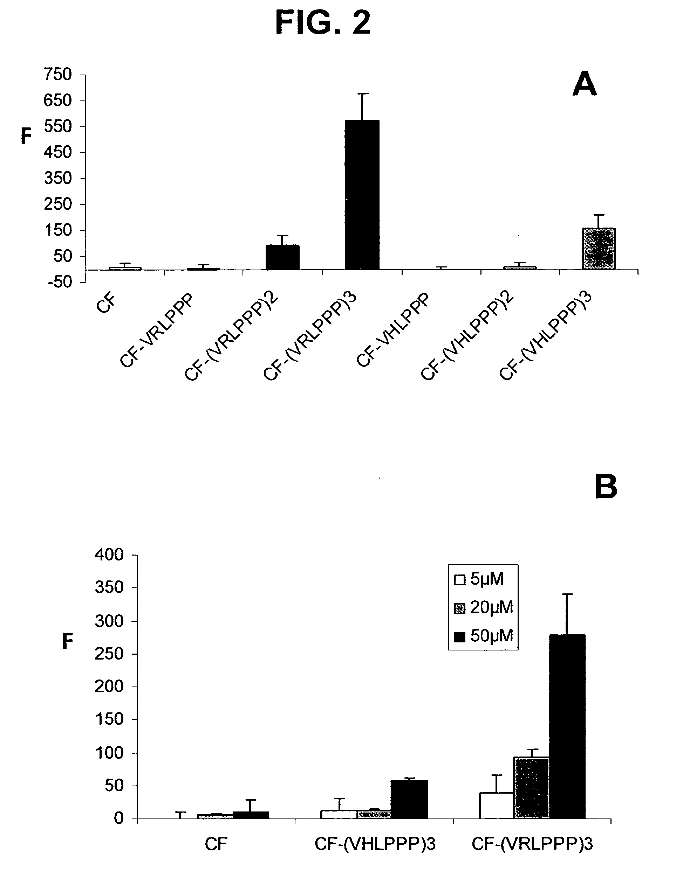

FIG. 2. shows the results of microplate fluorimeter reader assay. F means "fluorescence emission" in fluorescence units. FIG. 2A shows the fluorescence emitted after incubating HeLa cells for 3h with CF-(Val-X-Leu-Pro-Pro-Pro)ₙ with X = Arg and His and n = 1-3 at 50 µM concentration. FIG. 2B is a comparative representation of fluorescent emission obtained after incubation of HeLa cell line for 1 h at 37 °C with several carboxyfluoresceinated peptides at different concentrations ranging from 5 µM to 50 µM.

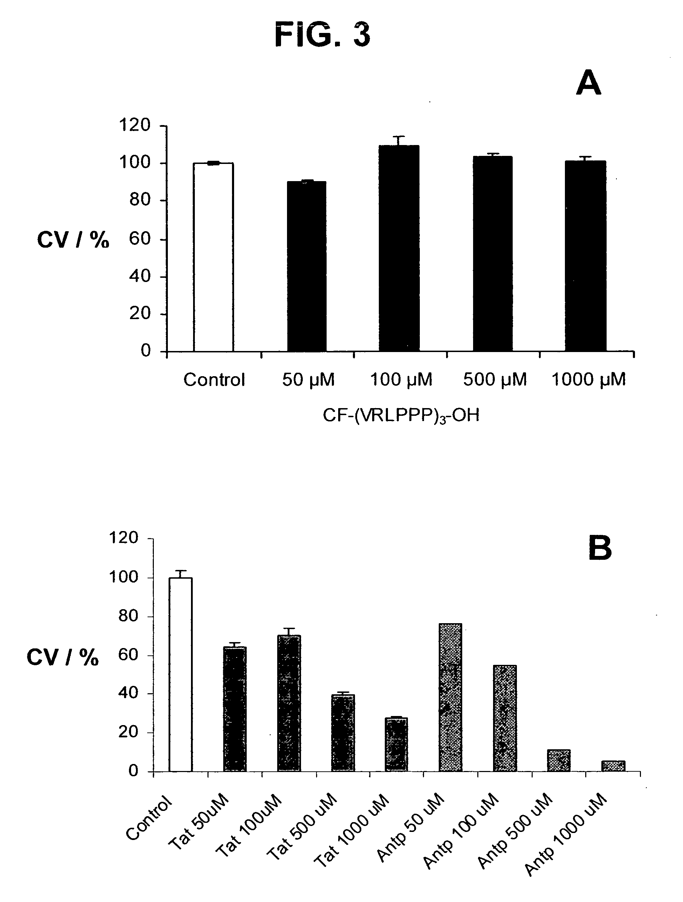

FIG. 3 shows the results of HeLa vilability and proliferation assay. CV means "cell viability" in %. In FIG. 3A cell viability was quantified by MTT staining, after 24h incubation of HeLa cells with the CF-(Val-Arg-Leu-Pro-Pro-Pro)₃ peptide at high concentration ranging from 50 to 1000 µM. In FIG. 3B HeLa viability was quantified by MTT staining after 24h incubation with CF-Tat-NH₂ and CF-Antp-NH₂ at a series of concentrations from 50 to 1000 µM.

### DETAILED DESCRIPTION OF PARTICULAR EMBODIMENTS

### Peptides Synthesis

(Val-His-Leu-Pro-Pro-Pro) based peptides were synthesized to be compared with (Val-Arg-Leu-Pro-Pro-Pro) based peptides. Synthesis of (Val-Arg-Leu-Pro-Pro-Pro)ₙ and (Val-His-Leu-Pro-Pro-Pro)ₙ, where n = 1-3, was performed by solid phase peptide synthesis on a 2-chlorotrityl resin. This support avoids the PP diketopiperazine side reaction. Compound 5(6)-carboxyfluorescein (CF) was used for the synthesis of the fluorescent labelled peptides required in the cell uptake studies. 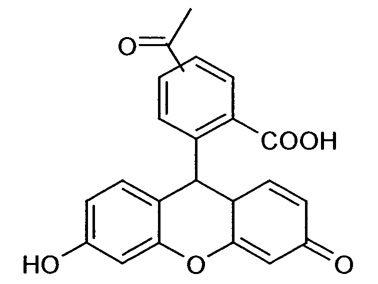 5(6)-carboxyfluorescein (CF)

Materials: Fmoc-protected amino acids were purchased from Advanced Chem Tech. Resin 2-chlorotrityl was obtained from CBL Patras. Coupling reagents: 7-azabenzotriazol-1-yloxytris(pyrrolidino)phosphonium hexafluorophosphate (PyAOP) was obtained from Applied Biosystems, rink amide MBHA resin and benzotriazol-1-yloxytris(pyrrolidino)phosphonium hexafluorophosphate (PyBOP) were purchased from Novabiochem, 1-hydroxy-7-azabenzotriazole (HOAt) was obtained from GL Biochem, 2-(1H-benzotriazol-1-yl)-1,1,3,3-tetramethyluronium tetrafluoroborate (TBTU) and 1-hydroxybenzotriazole (HOBt) were purchased from Albatros Chem Inc. Solvents: trifluoroacetic acid (TFA), piperidine, dimethylformamide (DMF), dichloromethane (DCM) and acetonitrile were purchased from SDS. 5(6)-carboxyfluorescein (CF) was obtained from Acros. Diisopropylcarbodimide (DIC) and N,N-diisopropylethylamine (DIEA) were obtained from Merck. Triisopropylsilane (TIS) was obtained from Fluka.

Peptides were synthesized according to standard protocols of solid phase peptide synthesis by using the 9-fluorenylmethoxycarbonyl/tert-butyl (Fmoc/tBu) strategy. Resin 2-chlorotrityl, Nα-Fmoc-protected amino acids (2 eq)/TBTU (2 eq) or PyBOP (2 eq) and DIEA (6 eq) were used. The syntheses of (Arg-Lys-Lys-Arg-Arg-Gln-Arg-Arg-Arg)-NH₂ (Tat) and (Arg-Gln-Ile-Lys-I le-Trp-Phe-Gln-Asn-Arg-Arg-Met-Lys-Trp-Lys-Lys)-NH₂ (Antp) were carried out automatically on an Applied Biosystems 433A Peptide Synthesizer. Rink Amide MBHA resin (0.1 mmol, 128 mg, initial loading of the resin = 0.78 mmol/g), Nα-Fmoc-protected amino acids (10 eq), TBTU/HOBt 0.45 M and DIEA 2 M in NMP were used for coupling reactions. As protecting groups for the side chains of Arg and His, 2,2,4,6,7-pentamethyldihydrobenzofurane-5-sulfonyl (Pbf) and tert-butyloxycarbonyl (Boc) were chosen. Cleavage of the Fmoc protecting group was carried out by treatment with a solution of 20% piperidine in DMF (2 x 10 min).

5(6)-carboxyfluorescein (CF) (5 eq), PyAOP (5 eq), HOAt (5 eq) and DIEA (10 eq) in DMF were dissolved in DMF/DCM 9/1, preactivated for 10 min and then added to the peptidyl-resin and stirred for 1 h 30 min. The labelled peptides were cleaved from the resin by treatment with 1% TFA in DCM for 5 min and the (Val-X-Leu-Pro-Pro-Pro)ₙ or CF-(Val-X-Leu-Pro-Pro-Pro)ₙ peptides (X is Arg or His) were obtained after treating the peptide-resin with 95% TFA, 2.5% TIS, 2.5% water from 1 h to 2h depending on the protecting group used for the side chains. A similar protocol was followed to obtain CF-Tat-NH₂ or CF-Antp-NH₂.

Peptides characterization: The labelled peptides were identified at λ = 443 nm on an analytical RP-HPLC [Waters 996 photodiode array detector equipped with Waters 2695 separation module, Symmetry^{®} column (C18, 5 µm, 4.6 x 150 mm) and a Millenium chromatography manager software; flow = 1ml/min; gradient = 5-100% B in 15 min (B = 0.036% TFA in acetonitrile)]. The carboxyfluoresceinated peptides were purified by semipreparative RP-HPLC [Waters 2487 Dual λ Absorbance Detector equipped with Waters 2700 Sample Manager, Waters 600 Controller, Waters Fraction Collector, Symmetry^{®} column (C18, 5 µm, 30 x 100 mm) and a Millenium chromatography manager software; flow = 10 ml/min; gradient = 5-20% D in 5 min; 20-70% D in 30 min; 70-100% D in 5 min (D = 0.1% TFA in acetonitrile)]. The target products were characterized by MALDI-TOF mass spectrometry (Vogayer-DE RP MALDI-TOF, PE Biosystems with a N₂ laser of 337 nm). Combination of experimental absorption values of the different carboxyfluoresceinated peptides obtained by UV at 490 and 443 nm and amino acid analysis permitted to obtain of the exact concentration for each sample (cf. TABLE 1).

### Cell culture and peptide treatments

Comparative properties of labelled peptides for translocation through the cell membrane were checked with HeLa human cell line. First, the extent of the uptake of the monomers, dimers and trimers at 50 µM concentration in HeLa cells was quantified using a fluorescence microplate reader assay. A dose-response analysis was then undertaken, incubating HeLa cells with the peptides at different concentrations. The cellular uptake of the new peptides was also studied by confocal laser scanning microscopy (CLSM).

HeLa cells were obtained from ATCC. They were maintained in DMEM (1000 mg/ml glucose) culture medium (Biological Industries) containing 10% Fetal Calf Serum (FCS), 2 mM glutamine, 50 u/ml penicillin and 0.05 g/ml streptomycin. For confocal microscopy experiments, exponentially growing HeLa cells were detached from the culture flasks using a trypsin-0.25% EDTA solution and the cell suspension was seeded at a concentration of 21.4 x 10³ cells/cm² onto glass coverslips or onto 8-well Lab-Teck^{™} chambered coverglass or on 96-well plate (Nalge Nunc International). Experiments were carried out 24h later, when the confluence was about 60-70%. The carboxyfluoresceinated compounds were dissolved in PBS and sterilized with 0.22 µm filters (Millex-GV, PVDF, Durapore, Millipore). The peptides and 5(6)-carboxyfluorescein stock solutions were diluted in the cell culture medium. Non-adherent cells were washed away and attached cells were incubated at 37 °C in CO₂ atmosphere in DMEM medium with a known concentration of peptides for 3h.

### Confocal Laser Scanning Microscopy (CLSM)

After 3h of incubation at 37 °C of HeLa cells with 5(6)-carboxyfluorescein (as negative control) or carboxyfluoresceinated peptides at 50 µM concentration, cells were rinsed for three times with PBS and fixed in a 3% p-formaldehyde solution for 15 min. Cells were then washed with PBS for 5 min and mounted with Mowiol mounting medium. CLSM was performed with a Leica SP2 confocal microscope. Images were taken using a 63X/1.3 NA oil immersion objective. As a fixation control, similar experiments were performed with cells plated onto glass bottom Lab-Tek^{™} chambers for live cell imaging. After incubation for 3 h, cells were washed for three times with PBS containing 1.1 mM CaCl₂ and 1.3 mM MgCl₂ and images were taken using a 60X/1.4 NA objective of an Olympus Fluoview 500 confocal microscope within the next 30 min. In both confocal microscopes, the carboxyfluorescein fluorescence was excited with the 488-nm line of an argon laser and its emission was detected in a range of 515-530 nm. The microscope settings were maintained identical for each peptide and dose.

Confocal microscopy images (images not shown in this description because of lack of color), revealed an intracellular vesicular distribution of the fluorescent peptides. The carboxyfluoresceinated peptides were located inside the cells and were not attached to the cell membrane. Influence of the fixation step with a 3% p-formaldehyde solution was examined, since the fixation step prior to observation by microscopy could lead to the presence of artefacts on entry, or otherwise could change the localization of the carrier molecule. A punctate cytoplasmic distribution outside the nucleus was observed in vivo and in fixed cells. Fixation with p-formaldehyde did not influence entry in HeLa cells and did not modify the localization of carrier peptides.

### Microplate fluorimeter reader assay

For each assay, 21.4 x 103 cells/cm² were seeded and cultured for 24 h. After complete adhesion to the plate, the culture medium was changed. Cells were incubated for 1 h or 3h at 37 °C under CO₂ atmosphere with fresh medium containing carboxyfluorescein-labelled peptides or carboxyfluorescein. Cells were washed with PBS. Cells were treated with lysis buffer (0.1 % Triton X-100 in 50 mM Tris, pH 8.5) for 30 min. The measurement of fluorescence emitted was carried out after 30 min incubation with lysis buffer in a FL600 Microplate Fluorescence Reader (Bio-Tek). Fluorescence was measured at a λ_{excitation} = 485/20 nm and a λₑₘᵢₛₛᵢₒₙ = 530/25 nm. A triplicate of each peptide or carboxyfluorescein concentration was performed and the fluorescence emitted for the blanks (cells with culture medium and without peptides) was subtracted. The experiment was reproduced three times.

As shown in FIG. 2A, cells incubated with CF-(Val-Arg-Leu-Pro-Pro-Pro)₃ presented a higher fluorescence intensity. The nature of the hydrophilic residues and, specifically, the length of the peptide were shown to have a pronounced effect on uptake (FIG. 2B).

### Peptides toxicity assay (MTT assay)

HeLa viability and proliferation in the presence of peptides was tested using a 3-(4,5-dimethylthiazol-2-yl)-2,5-diphenyltetrazolium bromide (MTT) assay. (cf. "MTT-cell proliferation assay" Cell Biology: A Laboratory Handbook, Academic Press, 1998, Second Ed. vol. 1; Y. Liu et al., "Mechanism of cellular 3-(4,5-dimethylthiazol-2-yl)-2,5-diphenyltetrazolium bromide (MTT) reduction", J. Neurochem. 1997 vol. 69 pp. 581-93).

For each assay 7 x 103 cells/cm² were seeded on a 96-well plate (Nange Nunc) (100 µl/hole) and cultured for 24 h. In order to avoid saturation in cell growth after 24h of peptide incubation, 7 x 103 cells/cm² were used in the proliferation measurement (instead of 21.4 x 103 cells/cm² seeded in previous experiments). After complete adhesion to the plate, the culture medium was discarded. Peptides were added at growing concentrations of 50, 100, 500, and 1000 µM. The cells were incubated for 3h or 24h at 37 °C under 5% CO₂ atmosphere. MTT was added 2h before the final incubation time, i.e., after 1 h for the 3h incubation time and after 22h for the 24h incubation time at a final concentration per hole of 0.5 mg/ml. The cells with peptide and MTT were incubated for a further 2h and then the medium was discarded by aspiration. Isopropanol was added to dissolve formazan, a dark blue coloured crystal observed in the wells. Absorbance was measured at λ = 570 nm, 30 min after the addition of isopropanol. The cell viability was expressed as a percent ratio of cells treated with peptide against cells untreated used as control.

Peptide CF-(Val-Arg-Leu-Pro-Pro-Pro)₃ was non-cytotoxic after incubation for 24h with HeLa cells in concentrations up to 1000 µM, which highlights its potential as a carrier (FIG. 3A). Comparative cytotoxic studies of other well established cell penetrating peptides have been done. CF-Tat-NH₂ and CF-Antp-NH₂ were cytotoxic at relatively low concentrations. At the concentration used for the internalization studies, i.e. 50 µM, CF-Tat-NH₂ reduced the cell viability to 64% and CF-Antp to 75% (FIG. 3B). The viability of HeLa cells was dramatically reduced in the presence of CF-Tat-NH₂ or CF-Antp-NH₂ at higher concentrations (e.g. to 40% and 11 % respectively at 500 µM). Measured against CF-Tat-NH₂ or CF-Antp-NH₂, the degree of internalization of CF-(Val-Arg-Leu-Pro-Pro-Pro)₃ was found to be respectively 15 or 20 times lower, however the latter showed the absence of cytotoxicity.

**TABLE 1. Characteristics of the synthesized peptides. All peptides have L-configuration amino acids.**

| | | | | |
|---|---|---|---|---|
| peptide sequences | reference | M (calculated) | M (found) | RT (min) Grad: 5-100% B |
| Val-His-Leu-Pro-Pro-Pro | RN 121322-14-3 | 659 | 660 [M+H⁺], | 4.82 |
| | SEQ ID NO: 1 | | 682 [M+Na⁺] | |
| CF-Val-His-Leu-Pro-Pro-Pro | | 1018 | 1018 [M+H⁺], | 7.46 |
| | | | 1040 [M+Na⁺] | |
| (Val-His-Leu-Pro-Pro-Pro)₂ | RN 132609-32-6 | 1297 | 1298 [M+H⁺], | 5.53 |
| | SEQ ID NO: 2 | | 1321 | |
| | | | [M+Na⁺], | |
| | | | 1337 [M+K⁺] | |
| CF-(Val-His-Leu-Pro-Pro-Pro)₂ | | 1657 | 1658 [M+H⁺] | 7.10 |
| (Val-His-Leu-Pro-Pro-Pro)₃ | RN 129460-93-1 SEQ ID NO: 3 | 1358 | 1359 [M+H⁺] | 5.84 |
| CF-(Val-His-Leu-Pro-Pro-Pro)₃ | | 1716 | 2299 [M+H⁺l | 6.9 |
| Val-Arg-Leu-Pro-Pro-Pro | SEQ ID NO: 4 | 678 | 679 [M+H⁺] | 4.8 |
| CF-Val-Arg-Leu-Pro-Pro-Pro | | 1019 | 1037 [M+H⁺] | 7.57 |
| (Val-Arg-Leu-Pro-Pro-Pro)₂ | SEQ ID NO: 5 | 1337 | 1338 [M+H⁺] | 5.55 |
| CF-(Val-Arg-Leu-Pro-Pro-Pro)₂ | | 1695 | 1696 [M+H⁺], | 7.24 |
| | | | 1718 [M+Na⁺] | |
| (Val-Arg-Leu-Pro-Pro-Pro)₃ | SEQ ID NO: 6 | 1997 | 1998 [M+H⁺] | 5.6 |
| CF-(Val-Arg-Leu-Pro-Pro-Pro)₃ | | 2356 | 2356 [M] | 7.02 |
| (Arg-Lys-Lys-Arg-Arg-Gln-Arg-Arg-Arg)-NH₂ | RN 130244-88-1 | 1337 | 1338 [M+H⁺] | 2.32 |
| | SEQ ID NO: 7 | | | |
| (Tat-NH₂) | | | | |
| CF-Tat-NH₂ | | 1697 | 1698 [M+H⁺], | 4.49 |
| | | | 1720 [M+Na⁺] | |
| (Arg-Gln-Ile-Lys-Ile-Trp-Phe-Gln-Asn-Arg-Arg-Met-Lys-Trp-Lys-Lys)-NH₂ | RN 214556-79-3 | 2244 | 2245 [M+H⁺], | 5.12 |
| | SEQ ID NO: 8 | | 2267 [M+Na⁺] | |
| (Antp-NH₂) | | | | |
| CF-Antp-NH₂ | | 2604 | 2605 [M+H⁺], | 6.12 |
| | | | 2626 [M+Na⁺] | |

## Claims

1. A compound of formula (I)
M₁-L₁-(Val-Arg-Leu-Pro-Pro-Pro)ₓ-L₂-M₂ (I)
or a pharmaceutically or a biologically acceptable salt thereof, wherein
Val is L-Val or D-Val;
Arg is L-Arg, D-Arg or L-(N-methyl)Arg;
Pro is L-Pro or D-Pro;
Leu is L-Leu or D-Leu;
x is an integer from 1 to 20;
L₁ and L₂ are chemical linkers, which are identical or different, present or absent;
M₁ and M₂ are pharmacologically and/or biologically active moieties, which are identical or different, present or absent; and
the bond between L₁ and M₁ and the bond between L₂ and M₂ are of any of the known chemical natures, including covalent and ionic.

2. The compound according to claim 1, wherein chemical linkers are independently selected from peptide sequences up to 10 amino acid residues.

3. The compound according to claim 1, wherein pharmacologically and/or biologically active moieties are active pharmaceutical ingredients, nucleic acids, peptide nucleic acids, peptides, proteins, carbohydrates, lipids, sterols, nanoparticles, antibodies, dyes or probes.

4. The compound according to any of the claims 1-3, wherein x is from 1 to 10.

5. The compound according to claim 4, wherein x is from 1 to 3.

6. The compound according to claim 5, wherein x is 3.

7. The compound according to claim 1, wherein L₁, L₂, M₁ and M₂ are all absent and, consequently the compound of formula (I) is
(Val-Arg-Leu-Pro-Pro-Pro)ₓ.

8. The compound according to claim 7, wherein x is 3.

9. The compound according to claim 7, having a sequence selected from the group consisting of SEQ ID NO: 1-6.

10. Use of the compound as defined in any of the claims 7-9 as cell penetrating carrier.

11. A pharmaceutical composition comprising a therapeutically effective amount of the compound defined in any of the claims 1-6, together with appropriate amounts of pharmaceutical excipients.

12. A cosmetic composition comprising a cosmetically effective amount of the compound defined in any of the claims 1-6, together with appropriate amounts of cosmetic excipients.
